# EUROPEAN PATENT APPLICATION

(11) **EP 3 838 346 A1**
(43) Date of publication of application: **23.06.2021**
(21) Application number: 19383177.3
(22) Date of filing: 20.12.2019
(51) Int. Cl.: A61P 29/02, A61K 9/48, A61K 31/164, A61K 47/22, A61K 47/44, A61P 15/00

(54) **SOFT GELATIN CAPSULES COMPRISING PALMITOYLETHANOLAMIDE AND ALPHA-LIPOIC ACID**

(71) Applicant: Chemo Research SL, 28050 Madrid (ES)
(72) Inventor: Rizo Martínez, José Miguel, 28043 MADRID (ES); Vicedo, Laura, 03680 ASPE (ES); Buzgau, Ioana Ramona, 19174 GALÁPAGOS (ES)
(74) Representative: ZBM Patents - Zea, Barlocci & Markvardsen

(57) **Abstract**

It is provided an oral pharmaceutical composition in the form of a soft gel capsule comprising a) an outer gelatin shell comprising gelatin, a plasticizer and water; and b) a filling composition comprising a therapeutically effective amount of a combination of palmitoylethanolamide and α-lipoic acid, together with a pharmaceutically acceptable carrier which is an oil. It is also provided a pharmaceutical composition as defined above for use in decreasing premenstrual or menstrual pain in women.

## Description

### Technical Field

The present invention is related to the field of soft gelatin capsules comprising pharmaceutical compositions. Particularly, it relates to a pharmaceutical composition comprising palmitoylethanolamide, to a soft gelatin capsule containing it, and to its use for the treatment of dysmenorrhea.

### Background Art

Dysmenorrhea, also known as menstrual pain, is pain during menstruation. Its usual onset occurs around the time that menstruation begins. Symptoms typically last less than three days. The pain is usually in the pelvis or lower abdomen. Dysmenorrhea is the leading cause of school or work absenteeism among adolescents and reproductive age women and it is strongly linked with positive scores for depression and anxiety. The most common pharmacological treatments for dysmenorrhea are nonsteroidal anti-inflammatory drugs (NSAIDs). The use of combined oral contraceptive pills (COCP) is another reasonable option.

Palmitoylethanolamide (PEA) belongs to the family of N-acylethanolamines (NAEs), endogenous biologically active lipids including the endogenous cannabinoid receptor ligand anandamide and the satiety factor oleoylethanolamide. Its IUPAC name is *N*-(2-hydroxyethyl)hexadecanamide and it structural formula is as follows:

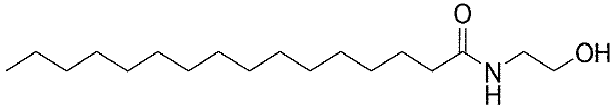

Preclinical and clinical studies suggest PEA may potentially be useful in a wide range of therapeutic areas, including eczema, pain and neurodegeneration. PEA has been demonstrated to bind to a receptor in the cell-nucleus (a nuclear receptor) and exerts a great variety of biological functions related to chronic pain and inflammation. PEA has been suggested to have useful analgesic properties while being devoid of unwanted effects.

In addition, PEA is available since 2005 as a supplement (nutraceutical). PEA could be considered an effective supplement to conventional analgesic therapies in the management of pelvic pain related to endometriosis, dysmenorrhea and dyspareunia. Thus, several clinical trials testing the efficacy of compositions comprising PEA in dysmenorrhea have been described (cf. Bouaziz, J. et al. in Cannabis and Cannabinoid Research, 2017, Vol. 2, No. 1, pp. 72-80).

Nevertheless, there is still the need of providing a stable oral pharmaceutical composition of PEA having a high effectiveness particularly for the treatment of dysmenorrhea.

### Summary of Invention

Inventors have found a stable pharmaceutical composition for oral administration containing PEA and α-lipoic acid. Although it is known the effect of PEA on menopausal pain relief, its combined use with lipoic has in a soft gelatine capsule has not been disclosed. By being administered in the form of soft gelatine capsules wherein the active ingredients are either dispersed or dissolved in oil, a faster absorption and higher bioavailability of the active ingredients is provided compared to other pharmaceutical forms. Thus, menstrual pain in women with dysmenorrhea is progressively decreased in a faster way.

α-Lipoic acid (LA) is a natural compound with antioxidant properties and other potential health benefits.

Thus, in a first aspect the present invention relates to an oral pharmaceutical composition in the form of a soft gelatine capsule comprising:
- an outer gelatin shell comprising gelatin, a plasticizer and water, and
- a filling composition comprising a therapeutically effective amount of a combination of palmitoylethanolamide and α-lipoic acid, together with a pharmaceutically acceptable carrier which is an oil.

Advantageously, the composition provides a fast and effective effect on the reduction of premenopausal and menopausal pain. Besides, soft gelatin capsules are easy to swallow, tasteless, and can have a wide variety of shapes, colours and sizes.

Additionally, it allows the combination of different active ingredients with different physical-chemical properties in the same pharmaceutical form, allowing the combination between powders and oils.

Thus, the oral pharmaceutical composition in the form of soft gelatine capsules comprising PEA and α-lipoic acid offers an improvement over the products available in the market, in the form of tablets, orodispersable sticks, hard capsules, or others.

The pharmaceutical composition of the invention can be used to progressively decrease menstrual pain. Accordingly, another aspect the invention relates to a pharmaceutical composition as defined herein above or below for use in decreasing premenstrual or menstrual pain in women. This aspect can also be formulated as the use of the pharmaceutical composition as defined herein above or below for the manufacture of a medicament for decreasing premenstrual or menstrual pain in women. It also relates to a method for decreasing premenstrual or menstrual pain in women comprising administering a therapeutically effective amount of the composition of the invention to a woman in need of the treatment.

### Detailed description of the invention

All terms as used herein in this application, unless otherwise stated, shall be understood in their ordinary meaning as known in the art. Other more specific definitions terms as used in the present application are as set forth below and are intended to apply uniformly throughout the specification and claims unless an otherwise expressly set out definition provides a broader definition.

The terms "particle size" and "particle size distribution", as used herein, are in terms of diameter irrespective of the actual particle shape. The term "diameter", as used herein, means the equivalent sphere diameter, namely the diameter of a sphere having the same diffraction pattern, when measured by laser diffraction, as the particle. Particle size can be determined, for example, by laser light scattering using a particle size analyser, such as a Mastersizer™ 2000 apparatus available from Malvern Instruments Ltd.

The expression "pharmaceutically acceptable" as used herein, means that the components so described are compatible with the other ingredients of the pharmaceutical composition, and are suitable for use in contact with human skin without undue toxicity, incompatibility, instability, allergic response, or the like.

The expression "therapeutically effective amount" as used herein, refers to the amount of a compound that, when administered, is sufficient to prevent the development of, or alleviate to some extent, one or more of the symptoms of the disorder or condition being treated. The particular dose of compound administered according to this invention will of course be determined by the particular circumstances surrounding the case, including the particular condition being treated, the duration of the treatment, the nature of any concurrent treatment, and any other factor known to the expert.

The term "Bloom number" refers to a measure of the gel strength of gelatin, reflecting the average molecular weight of its constituents. The higher the Bloom number the stiffer the gelatin.

The term "wt. %" refers to the percentage by weight of the ingredient per weight of the overall composition, unless otherwise stated.

As used herein, the indefinite articles "a" and "an" are synonymous with "at least one" or "one or more". Unless indicated otherwise, definite articles used herein such as "the" also include the plural of the noun.

As mentioned above, the oral pharmaceutical composition in the form of a soft gel capsule of the invention comprises an outer gelatin shell and a filling composition comprising PEA, α-lipoic acid, and a pharmaceutically acceptable oil.

In an embodiment of the oral pharmaceutical composition in the form of a soft gel capsule of the present invention, the oil is selected from the group consisting of soy, peanut, sunflower, wheat germ, evening primrose oil, fish oil, olive oil, coconut, corn oil, cotton seed oil, palm oil, sesame oil, algae oil, pumpkin oil, chia oil, borage, safflower oil, and linseed oil. Particularly, the oil is sunflower oil or a mixture of sunflower oil and evening primrose oil.

Gamma-Linolenic Acid (GLA), part of the omega-6 family, is found in evening primrose, black currant and borage oils. Evening primrose oil is obtained from the seeds of the evening primrose plant (*Oenothera biennis*). GLA, especially in the form of evening primrose oil, is well known for its role in providing nutritional support for women with PMS (premenstrual syndrome). The body converts GLA to a hormone-like substance called prostaglandins which control virtually every organ in the body. These compounds especially affect the heart and circulation, skin, immunity, and inflammation. Evening primrose oil contains both linoleic acid (74%) and GLA (9%), making it the most familiar and popular source of GLA. Under normal conditions, the body uses linoleic acid to produce GLA.

Sunflower oil is the non-volatile oil pressed from the seeds of sunflower (*Helianthus annuus*). It is a mixture mainly of the polyunsaturated fat linoleic acid (59% of total) and the monounsaturated fat oleic acid (30% of total).

In an embodiment, optionally in combination with one or more features of the particular embodiments defined above, the filing composition of the soft gel capsule further comprises vitamin C, and vitamin D3.

Vitamin C, also known as L-ascorbic acid, is a naturally occurring organic compound with antioxidant properties found in various foods and sold as a dietary supplement. Studies suggest that taking vitamin C daily and increasing it around the time of the menstrual cycle, might lessen premenstrual syndrome symptoms and ease heavy bleeding during our periods.

Vitamin D3, also known as cholecalciferol, is a type of vitamin D which is made by the skin when exposed to sunlight; it is also found in some foods and can be taken as a dietary supplement. The use of Vitamin D supplementation to improve pain intensity and decrease the need for using NSAID in patients with primary dysmenorrhea and vit D deficiency has been disclosed.

It has been found that the addition of vitamin C and vitamin D3 to a composition comprising PEA and lipoic acid has an improved effect on the reduction of menopausal pain. This effect is even better in the presence of linoleic acid (contained in the sunflower oil), and, optionally, of gamma-linolenic acid (contained in the primrose oil).

A soft gelatin capsule (also referred to as soft elastic gelatin capsules, liquid gels or softgels) is a hermetically sealed, one-piece capsule (outer gelatin shell) surrounding a liquid or semi-solid center fill.

Soft gelatin capsules are easy to swallow, tasteless, can have a variety of shapes, colours and sizes.

Gelatin is an essential component of the soft gelatin shells. The starting gelatin material used in the manufacture of soft capsules is obtained by the partial hydrolysis of collagenous material, such as the skin, white connective tissues, or bones of animals. Gelatin material can be classified as Type A gelatin, which can be obtained from the acid-processing of porcine skins, and Type B gelatin, which can be obtained from the alkaline-processing of bone and animal (bovine) skins. Gelatin from pig and bovine origin are preferably used for their gelling, film forming properties.

In an embodiment, optionally in combination with one or more features of the particular embodiments defined above, the shell of the soft gelatin capsules of the present disclosure comprises from 20 wt% to 60 wt% gelatin, particularly from 25 wt% to 50 wt% gelatin, more particularly from 40 wt% to 50 wt% gelatin, based on the total weight of gelatin, plasticizer, water, and carotene. The gelatin can be Type B, or a mixture of Type A and Type B with bloom numbers ranging from about 150 to about 200.

The Bloom test is a test to measure the strength of a gel or gelatin. The test determines the weight in grams needed by a specified plunger (normally with a diameter of 0.5 inch) to depress the surface of the gel by 4 mm without breaking it at a specified temperature. The number of grams is called the Bloom number, and most gelatins are between 30 and 300 g Bloom. The higher a Bloom number, the higher the melting and gelling points of a gel, and the shorter its gelling times. The method is most often used on soft gels. To perform the Bloom test on gelatin, a 6.67% gelatin solution is kept for 17-18 hours at 10 °C prior to being tested.

As mentioned above, a plasticizer is another component of the soft gelatin shell. One or more plasticizers can be incorporated to produce a soft gelatin shell. Examples of useful plasticizers include glycerin, sorbitan, sorbitol, or similar low molecular weight polyols, and mixtures thereof.

In an embodiment, optionally in combination with one or more features of the particular embodiments defined above, the shell of the soft gelatin capsules of the present disclosure comprises from 10 wt% to 35 wt% plasticizer, particularly from 10 wt% to 25 wt% plasticizer, and more particularly from 10 wt% to 20 wt% plasticizer, based on the total weight of gelatin, plasticizer, water, and carotene. In a particular embodiment, the plasticizer is glycerin.

The soft gelatin shells also comprise water. The water is believed to aid in the rapid dissolution or rupture of the soft gelatin shell upon contact with the gastrointestinal fluids. In an embodiment, optionally in combination with one or more features of the particular embodiments defined above, the shell of the soft gelatin capsules of the present disclosure comprises from 15% to 50% water, particularly from 25% to 40% water, and most preferably from 30% to 40% water, based on the total weight of gelatin, plasticizer, water, and carotene.

Carotenes can also be incorporated into the soft gelatin shell. Carotenes are used as dyes, opacifying the soft gelatin capsule. The amount of carotenes such as of beta-carotene can be from 1 wt% to 2 wt%, based on the total weight of gelatin, plasticizer, water, and carotene.

In another embodiment, optionally in combination with one or more features of the particular embodiments defined above, the shell of the soft gelatin capsules of the present disclosure comprises from 40 wt% to 50 wt% gelatin, from 10 wt% to 20 wt% plasticizer, from 30% to 40% water, and from 1 wt% to 2 wt% beta-carotene, based on the total weight of gelatine, plasticizer, water and beta-carotene.

The active ingredient PEA can be synthesized by reaction of palmitic acid in presence of ethanolamine, purification and crystallization, although it is also commercially available.

In an embodiment of the pharmaceutical composition of the present disclosure, optionally in combination with one or more features of the particular embodiments defined above, PEA is in an amount ranging from 100 to 450 mg, particularly in an amount of 400 mg.

In soft capsule PEA is suspended in the oil carrier.

In another embodiment of the pharmaceutical composition of the present disclosure, optionally in combination with one or more features of the particular embodiments defined above, Vitamin D3 is in an amount ranging from 0.01 mg to 12 mg, particularly from 0.02 to 10 mg, more particularly from 0.1 mg to 0.5 mg.

Additionally, the filling composition can also comprise a thickener such as beeswax, glyceryl monostearate and mixtures thereof. Particularly, the thickener is beeswax.

The capsules of the present invention can be prepared according to procedures well known in the art normally used to prepare soft gelatin capsules, such as by the Rotary Die Process (cf. Raj, A. "Soft Gelatin Capsules (Softgels)" PharmaTutor2015, Vol. 3, pp.16-18).

As mentioned above, it has been proven through clinical studies that the PEA in combination with the active ingredients of the formula, progressively decreases menstrual pain, which allows avoiding the use of anti-inflammatory drugs.

Throughout the description and claims the word "comprise" and variations of the word, are not intended to exclude other technical features, additives, components, or steps. Furthermore, the word "comprise" encompasses the case of "consisting of". The following examples and drawings are provided by way of illustration, and they are not intended to be limiting of the present invention. Furthermore, the present invention covers all possible combinations of particular and preferred embodiments described herein.

### Examples

### Example 1 - Soft gel capsule

A soft gel capsule comprising the composition as shown in the Table 1 below was prepared:

**Table 1**

| **Filling composition** | **mg/capsule** |
|---|---|
| Sunflower Oil | 216.06 |
| Evening Primrose Oil | 500.00 |
| PEA | 404.04 |
| α-Lipoic Acid | 10.00 |
| Vitamin C | 12.00 |
| Vitamin D3 | 0.20 |
| **Total filling** | **1,142.30** |

| **Shell composition** | **mg/cap** |
|---|---|
| Gelatin | 298,86 |
| Glycerin 100% USP | 137,40 |
| Beta-carotene | 13,74 |
| **Total Wet Shell** | **450.00** |

### Manufacturing process

PEA was provided by FrauPharma (Italy) as a fine crystalline powder with a particle size D50 <50 µm (laser diffraction). The particle size distribution of two analysis:

| | |
|---|---|
| D₁₀ = 8.823 µm | D₁₀ = 8.043 µm |
| D₅₀ = 37.370 µm | D₅₀ = 32.385 µm |
| D₉₀ = 93.485 µm | D₉₀ = 81.309 µm |
| D_{4,3} = 45,089 | D_{4,3} = 39.296 |

Particle size was determined by laser light scattering using a Malvern Mastersizer™ 2000 (Malvern Instruments Ltd.).

### 1. Gelatin shell mixture preparation.

In a reactor surrounded by a thermal jacket, gelatin was mixed with glycerin and water, and the mixture was heat while stirring with a very high torque tribune mixer under vacuum. Then, β-carotene as a colourant agent was added.

### 2. Preparation of the filling composition:

In a reactor, sunflower oil and primrose oil were mixed at 25-30 °C. Subsequently, PEA, lipoic acid and vitamins were added, and the mixture was stirred until homogenization.

### 3. Preparation of the soft gel capsules

The gelatin shell mixture and the filling composition were mixed in a soft gel capsule machine to form soft gel capsules, which were subsequently dried and packaged.

### Example 2 - Soft gel capsule

Similarly as in Example 1, a soft gel capsule comprising the composition as shown in the Table 2 below was prepared:

**Table 2**

| **Filling composition** | **mg/capsule** |
|---|---|
| Sunflower Oil | 730.00 |
| PEA | 400.00 |
| α-Lipoic Acid | 10.00 |
| **Total filling** | **1,140.00** |

| **Shell composition** | **mg/cap** |
|---|---|
| Gelatin | 298,86 |
| Glycerin 100% USP | 137,40 |
| Beta-carotene | 13,74 |
| **Total Wet Shell** | **450.00** |

### Example 2 - Efficacy of PEA soft gel capsule on dysmenorrhea

In order to assess the efficacy of a soft gel capsule containing palmitoylethanolamide (PEA) and α-lipoic acid on dysmenorrhea, a double-blind, multiple dose, randomized and two-sequence/two-period crossover clinical trial (2x2 crossover design), with balanced carry-over effect compared with placebo was carried out.

The population for the study was 36 women with an age range of 18-45 inclusive. Each subject took three capsules of 400 mg PEA or three capsules of 400 mg placebo.

To evaluate the efficacy of the treatment, a determination of menstrual pain intensity measure (VAS score) is performed. For this purpose, each participant completed a VAS 72 hours after the start of each menstrual cycle VAS scale (at 24 h, 48 h, and 72 h).

The safety assessment was determined by:
- Recording of possible adverse events during the course of the study.
- Vital signs (BP, HR and RR).
- ECG recording (selection visit (V0) and V7).
- Evaluation of analytical (hematological and biochemical) results.

Each subject took 135 gelatin capsules of PEA 400 mg in one sequence and 135 gelatin capsules of placebo in the other sequence. 45 capsules at each menstrual cycle. During the 5 days before and the 10 days after (15 days), the start of menstrual bleeding at each cycle, the subject took three capsules a day, one with each main meal (breakfast, lunch and dinner). In the event that a subject did not take a dose on a particular day, he did not take another additional dose the next day.

Capsule formulation used for the administration of the study treatments is the capsule of Example 2 (PEA) and the one of Table 3 below for placebo:

**Table 3**

| **Filling composition** | **mg/capsule** |
|---|---|
| Sunflower Oil | 1130.00 |
| α-Lipoic Acid | 10.00 |
| **Total filling** | **1,140.00** |

| **Shell composition** | **mg/cap** |
|---|---|
| Gelatin | 298,86 |
| Glycerin 100% USP | 137,40 |
| Beta-carotene | 13,74 |
| **Total Wet Shell** | **450.00** |

To ensure statistical validity in the development of the study, both in the selection and distribution of the groups and in the interpretation of the results, the analysis of the data is performed using the SPSS software for Windows.

The descriptive and graphical presentation of the data is performed using means and/or medians as a measure of the central tendency of the values of the variables measured in the study. For this, the mean or median values of each of the objective parameters in each experimental group is determined at the beginning, and in the different sequences of the study. The corresponding measures of dispersion (standard deviation or interquartile range) is calculated both in front of the previous visit and in front of the baseline visit. The study variables is compared in women before and after the intake of the study treatment (PEA) and before and after intake of placebo with the Wilcoxon nonparametric test. The effect of both treatments is compared with a covariance analysis (ANCOVA). In all hypothesis tests, the null hypothesis is rejected when the value of p is less than 0.05. That is, significant differences are considered if α or type I error is <0.05.

Results are shown in tables 4, 5, and 6 below.

**Table 4**

| 24 h | | Cycle 1 | Cycle 2 | Cycle 3 |
|---|---|---|---|---|
| PEA | Mean | 5.58 | 4.88 | 4.38 |
| | Median | 5.80 | 5.00 | 3.80 |
| PLACEBO | Mean | 4.99 | 5.53 | 5.58 |
| | Median | 5.70 | 6.30 | 6.65 |
| Difference (PEA vs PL) | | 0.59 | -0.65 | -1.20 |
| | | 0.10 | -1.30 | -2.85 |

| 48 h | | Cycle 1 | Cycle 2 | Cycle 3 |
|---|---|---|---|---|
| PEA | Mean | 2.71 | 3.39 | 3.23 |
| | Median | 2.50 | 4.00 | 1.40 |
| PLACEBO | Mean | 3.68 | 3.05 | 3.97 |
| | Median | 3.95 | 2.80 | 3.40 |

| 24 h | | Cycle 1 | Cycle 2 | Cycle 3 |
|---|---|---|---|---|
| Difference (PEA vs PL) | | -0.97 | 0.34 | -0.74 |
| | | -1.45 | 1.20 | -2.00 |

| 72 h | | Cycle 1 | Cycle 2 | Cycle 3 |
|---|---|---|---|---|
| PEA | Mean | 1.43 | 1.23 | 1.42 |
| | Median | 0.20 | 0.20 | 0.50 |
| PLACEBO | Mean | 1.65 | 0.64 | 1.88 |
| | Median | 0.80 | 0.00 | 0.75 |
| Difference (PEA vs PL) | | -0.22 | 0.60 | -0.46 |
| | | -0.60 | 0.20 | -0.25 |

**Table 5 - PEA Group. First VAS record (24 h post dose)**

| | Mean | Median | Median Diff |
|---|---|---|---|
| Cycle 1 | 5.58 | 5.8 | |
| Cycle 2 | 4.88 | 5 | 0.8 |
| Cycle 3 | 4.38 | 3.8 | 1.2 |
| | | | 2 |

**Table 6 - Placebo Group. First VAS record (24 h post dose)**

| | Mean | Median | Median Diff |
|---|---|---|---|
| Cycle 1 | 4.99 | 5.7 | |
| Cycle 2 | 5.53 | 6.3 | -0.6 |
| Cycle 3 | 5.58 | 6.65 | -0.35 |
| | | | -0.95 |

As can be seen from the data above, PEA is not an acute analgesic but has a maintained activity. It was observed, in the evolution of pain intensity at 24 hours when patients took PEA, that the mean/median values have reduced from 5.58/5.80 in cycle 1 to 4.38/3.80 in cycle 3 (more than 1 unit). This evolution was not observed with the placebo: PEA reduced pain at 24h of cycles and as the number of cycles taking PEA increased the decrease in pain was greater.

These results indicate that with the administration of PEA for 15 days (5 days before and the 10 days after the start of menstrual bleeding) at each cycle, as an adjuvant to the usual analgesic, reduces the pain threshold in consecutive menstrual cycles and facilitates analgesic action of another pain reliever, such as NSAIDs or paracetamol.

### Citation List

1. Bouaziz, J. et al. in Cannabis and Cannabinoid Research, 2017, vol. 2, pp. 72-80.
2. Raj, A. "Soft Gelatin Capsules (Softgels)" PharmaTutor2015, Vol. 3, pp.16-18.

## Claims

1. An oral pharmaceutical composition in the form of a soft gelatine capsule comprising:
- an outer gelatin shell comprising gelatin, a plasticizer and water, and
- a filling composition comprising a therapeutically effective amount of a combination of palmitoylethanolamide and α-lipoic acid, together with a pharmaceutically acceptable carrier which is an oil.

2. The oral pharmaceutical composition in the form of a soft gel capsule of claim 1, wherein the oil is selected from the group consisting of soy, peanut, sunflower, wheat germ, evening primrose oil, fish oil, olive oil, coconut, corn oil, cotton seed oil, palm oil, sesame oil, algae oil, pumpkin oil, chia oil, borage, safflower oil, and linseed oil.

3. The oral pharmaceutical composition in the form of a soft gel capsule of claim 2, wherein the oil is sunflower oil or a mixture of sunflower oil and evening primrose oil.

4. The oral pharmaceutical composition in the form of a soft gel capsule of any one of claim 1 to 3, wherein the filing composition further comprises vitamin C, and vitamin D3.

5. The oral pharmaceutical composition in the form of a soft gel capsule of any one of claim 1 to 4, wherein the gelatin shell comprises from 20 wt% to 60 wt% gelatin based on the total weight of gelatin, plasticizer, water, and carotene.

6. The oral pharmaceutical composition in the form of a soft gel capsule of any one of claim 1 to 5, wherein the gelatin has a Bloom number ranging from 150 to 200.

7. The oral pharmaceutical composition in the form of a soft gel capsule of any one of claims 1 to 6, wherein the gelatin shell comprises from 10 wt% to 35 wt% plasticizer based on the total weight of gelatin, plasticizer, water, and carotene.

8. The oral pharmaceutical composition in the form of a soft gel capsule of any one of claims 1 to 7, wherein the plasticizer is selected from the group consisting of glycerin, sorbitan, sorbitol, and mixtures thereof.

9. The oral pharmaceutical composition in the form of a soft gel capsule of any one of claims 1 to 8, wherein the gelatin shell comprises from 15 wt% to 50 wt% water based on the total weight of gelatin, plasticizer, water, and carotene.

10. The oral pharmaceutical composition in the form of a soft gel capsule of any one of claims 1 to 9, wherein the gelatin shell comprises from 40 wt% to 50 wt% gelatin, from 10 wt% to 20 wt% plasticizer, from 30% to 40% water, and from 1 wt% to 2 wt% beta-carotene, based on the total weight of gelatine, plasticizer, water and beta-carotene.

11. The oral pharmaceutical composition in the form of a soft gel capsule of any one of claims 1 to 10, wherein PEA is in an amount ranging from 100 to 450 mg, particularly in an amount of 400 mg.

12. The oral pharmaceutical composition according to any one of claims 1 to 11 for use in decreasing premenstrual or menstrual pain in women.
